# EUROPEAN PATENT APPLICATION

(11) **EP 2 821 005 A1**
(43) Date of publication of application: **07.01.2015**
(21) Application number: 13754325.2
(22) Date of filing: 27.02.2013
(51) Int. Cl.: A61B 1/31, A61B 1/012

(54) **AUXILIARY TOOL FOR COLONOSCOPY**

(30) Priority: 29.02.2012 KR 20120020717
(71) Applicant: The Catholic University of Korea Industry-Academic Cooperation Foundation, Seoul 137-701 (KR)
(72) Inventor: KANG, Sang Yun, Daegu 706-828 (KR)
(74) Representative: Mounteney, Simon James
(86) International application number: PCT/KR2013/001558
(87) International publication number: WO 2013/129827

(57) **Abstract**

An auxiliary tool for colonoscopy is disclosed. The auxiliary tool for colonoscopy includes an anal intubation body and a feces-holding body. The anal intubation body has an aperture surface on one side and an anus cover on the other side, and also has a hollow part having a first diameter formed inside. The anus cover is shaped as a cap having a round curved surface. The feces-holding body has one side coupled to an inner side of the anus cover, has a holding part formed inside, and has a leakage prevention body formed on the other side. The holding part has a second diameter and is connected with the hollow part, and the leakage prevention body is configured to permit passage of an endoscope tube but prevent leakage of fecal matter held in the holding part to the other side. The auxiliary tool for colonoscopy can prevent damage to the anus during colonoscopy and guarantee a field of vision around the anal verge to allow accurate observation and diagnosis over an area extending from the anal verge to the lower rectum.

## Description

### 1. Technical Field

The present invention relates to an auxiliary tool for colonoscopy.

### 2. Relaed Art

The digestive organs of the human body includes the esophagus, stomach, small intestine, and large intestine. The large intestine, which forms the final part of the digestive tract, is where the absorption of water and electrolytes mostly occurs. The large intestine can be divided mainly into the colon and the rectum, where the colon can in turn be divided into the ascending colon, the transverse colon, the descending colon, and the sigmoid colon. Cancer occurring on the colon is referred to as colon cancer, cancer occurring on the rectum is referred to as rectal cancer, and these two types are collectively referred to as bowel cancer or colorectal cancer.

Causes of colorectal cancer can be largely divided into environmental causes and genetic causes, but intake of high calories, intake of animal fats, lack of fiber intake, obesity, etc., are known to be particularly relevant to colorectal cancer.

Types of examination methods that can help to diagnose colorectal cancer include digital rectal examination, fecal examination, colorectal screening, CT or MRI scanning, ultrasonic scanning, blood examination, etc., but a definite diagnosis of colorectal cancer is possible only when cancer cells are discovered through a biopsy entailing colonoscopy. Thus, colonoscopy is widely used for diagnosing colorectal cancer and other related diseases.

Colonoscopy is an examination method in which an endoscope is inserted through the anus to observe and photograph the inside of the colon. An endoscope in current use may be a tool having a length of 1.3 - 1.7 m, with a camera connected by an optical fiber attached to the end of a flexible tube.

Colonoscopy is frequently used in diagnosing and treating colorectal cancer and inflammatory bowel diseases, as it enables diagnosis by direct observation of the inside of the large intestine, as well as treatment procedures such as for hemostasis, tissue biopsy, or removing suspicious lesions.

In order to obtain an accurate field of vision for increased accuracy in diagnosis or treatment using colonoscopy, a patient is required, before the examination, to undergo a bowel cleansing procedure, which may involve taking an enema mixed with large amounts of water and defecating several times. This procedure can be fatiguing and inconvenient. Also, the endoscope tube having a diameter of about 1.8 cm may, despite its flexibility, cause scars in the anal mucosa as it passes through and causes friction at the anus during the colonoscopy. This can result in a patient suffering pain during defecation for several days afterward.

Also, the duration of a colonoscopy examination can be delayed depending on bowel resiliency, which in turn may depend on the patient's age, bowel adhesion, which may depend on the patient's history of abdominal surgery, and other anatomical differences of the intestine. In such cases, there may be increased pain resulting from the procedure, even when sleep anesthesia is employed.

During colonoscopy, the endoscope moves in a direction opposite to the direction of bowel movement, and since the inside of the colon is observed after inserting the endoscope up to the cecum while injecting a suitable amount of air into the colon and sucking in serous fluid and foreign bodies, the gases within the colon together with liquids in the colon would frequently spurt out between the anus and the endoscope during the examination, causing much embarrassment for the patient. Often, patients would refrain from taking colonoscopy examinations, which should be taken regularly, because of such complications during defecation, embarrassment, or fear of the colonoscopy procedure, whereby patients would miss the opportunity to find and treat colorectal cancer in its early stages.

At the opening of the anus is the anal verge. It may be difficult to accurately observe areas around the anal verge or areas from the anal verge to the lower rectum using a camera mounted on the end of the endoscope tube and connected with an optical fiber, because during colonoscopy, the anus closes due to the resiliency of the anus when the endoscope is inserted through the anus, and because the field of vision is clouded by stool matter from the anal verge to the lower rectum. Also, in order to diagnose a disease occurring at the anal verge or the lower rectum using an endoscope, the endoscope inserted inside the colon must be turned around inside the intestine in the reverse direction, but this creates a risk of puncturing a wall of the intestine. As such, there are many difficulties to consider when using colonoscopy for diagnosis on the anal verge or the lower rectum.

In relation to the above, Korean Patent Publication No. 10-2008-0084105 discloses an anal adapter for an endoscope which uses air balloons to firmly secure the adapter, prevent gases or fecal matter from leaking out by sealing the gaps between the endoscope and the adapter, and prevent damage to the anus by preventing the endoscope from directly contacting the anus.

### SUMMARY

An aspect of the invention is to provide an auxiliary tool for colonoscopy that can prevent damage to the anus during colonoscopy and guarantee a field of vision around the anal verge to allow accurate observation and diagnosis over an area extending from the anal verge to the lower rectum.

One aspect of the invention provides an auxiliary tool for colonoscopy that includes an anal intubation body and a feces-holding body. The anal intubation body has an aperture surface on one side and an anus cover on the other side, and also has a hollow part having a first diameter formed inside. The anus cover is shaped as a cap having a round curved surface. The feces-holding body has one side coupled to an inner side of the anus cover, has a holding part formed inside, and has a leakage prevention body formed on the other side. The holding part has a second diameter and is connected with the hollow part, and the leakage prevention body is configured to permit passage of an endoscope tube but prevent leakage of fecal matter held in the holding part to the other side.

The second diameter can be greater than the first diameter.

The leakage prevention body can include a first leakage prevention sub-body, which may include a first radial leakage prevention membrane that includes a multiple number of first cut pieces cut in a radial arrangement, and a first ring body having the first radial leakage prevention membrane coupled to an inner portion thereof.

The first leakage prevention sub-body can further include a second radial leakage prevention membrane disposed between the first radial leakage prevention membrane and the first ring body, where the second radial leakage prevention membrane can include a multiple number of second cut pieces cut in a radial arrangement, and where the second radial leakage prevention membrane can be coupled in an overlapping manner with the first radial leakage prevention membrane inside the first ring body such that the second cut pieces are positioned directly underneath slits between the first cut pieces.

The leakage prevention body can further include a second leakage prevention sub-body, which may include a circular leakage prevention membrane and a second ring body. Here, an endoscope through-hole having a diameter smaller than an outer diameter of the endoscope tube can be formed in the circular leakage prevention membrane, and the circular leakage prevention membrane can be fitted into an inner portion of the second ring body.

A valve-connecting part can be formed on a perimeter of the feces-holding body, and the auxiliary tool can further include an open/shut valve, which may be installed between a suction device and the valve-connecting part such that it is capable of opening and shutting, and which may be configured to control a flow of fecal matter according to a manipulation of an open/shut handle. Here, the open/shut valve can include a socket, in which an inlet connected to the valve-connecting part and an outlet connected to the suction device may be formed, a cylindrical valve body, in which a through-hole connecting to the inlet and the outlet may be formed, and which may be coupled to the socket such that it is capable of changing direction to open and shut the inlet and the outlet, and an open/shut handle, which may be connected to the cylindrical valve body and be configured to change a direction of the cylindrical valve body according to a manipulation.

At least one of the anal intubation body and the feces-holding body can be made from a transparent synthetic resin material.

Additional aspects and advantages of the present invention will be set forth in part in the description which follows, and in part will be obvious from the description, or may be learned by practice of the invention.

According to an embodiment of the invention, damage to the anus can be avoided during a colonoscopy examination, so that the discomfort felt by the patient after the colonoscopy can be minimized.

Also, since it is possible to form an airtight seal, the colon can be expanded with a minimal amount of air, reducing any pain caused by excessive air injection, and delays in colonoscopy caused by the formation of loops can be reduced.

Even after the preparatory procedures for colonoscopy, fecal matter such as stool, etc., may remain within the colon, but such fecal matter can be prevented from leaking out.

Also, in the case of an elderly patient having anal sphincter atony or degraded functioning of the anal sphincter, it can be difficult to observe the colon, especially the rectum area, due to air leaking out through the anus. However, the auxiliary tool for colonoscopy based on an embodiment of the invention can prevent the air from leaking and can thus be utilized with greater ease, for example when removing polyps in the rectum.

When observing the anal verge area, the endoscope may have to be reversed inside the intestine. By using the auxiliary tool that has a transparent body, it is possible to provide an accurate field of vision around the anal verge area, obviating the need to turn the endoscope in the reverse direction. Since it is possible to adjust the depth by which the tool body is inserted through the anus, the examination can be performed evenly over the anal verge.

### BRIEF DESCRIPTION OF THE DRAWINGS

Figure 1 is a perspective view of an auxiliary tool for colonoscopy according to an embodiment of the invention,
Figure 2 is an exploded perspective view of an auxiliary tool for colonoscopy according to an embodiment of the invention,
Figure 3A and Figure 3B illustrate a first radial leakage prevention membrane and a second radial leakage prevention membrane coupled together,
Figure 4 illustrates the leakage prevention structure of a first radial leakage prevention membrane and a second radial leakage prevention membrane,
Figure 5 illustrates an example of utilizing an auxiliary tool for colonoscopy according to an embodiment of the invention.

### DETAILED DESCRIPTION

As the present invention allows for various changes and numerous embodiments, particular embodiments will be illustrated in the drawings and described in detail in the written description. However, this is not intended to limit the present invention to particular modes of practice, and it is to be appreciated that all changes, equivalents, and substitutes that do not depart from the spirit and technical scope of the present invention are encompassed in the present invention. In describing the invention, detailed descriptions of known technology are omitted when they are deemed to obscure the essence of the invention.

While such terms as "first" and "second," etc., may be used to describe various components, such components must not be limited to the above terms. The above terms are used only to distinguish one component from another.

The terms used in the present specification are merely used to describe particular embodiments, and are not intended to limit the present invention. An expression used in the singular encompasses the expression of the plural, unless it has a clearly different meaning in the context. In the present specification, it is to be understood that terms such as "including" or "having," etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

An embodiment of the invention will be described below in detail with reference to the accompanying drawings. Those components that are the same or are in correspondence are rendered the same reference numeral regardless of the figure number, and redundant descriptions are omitted.

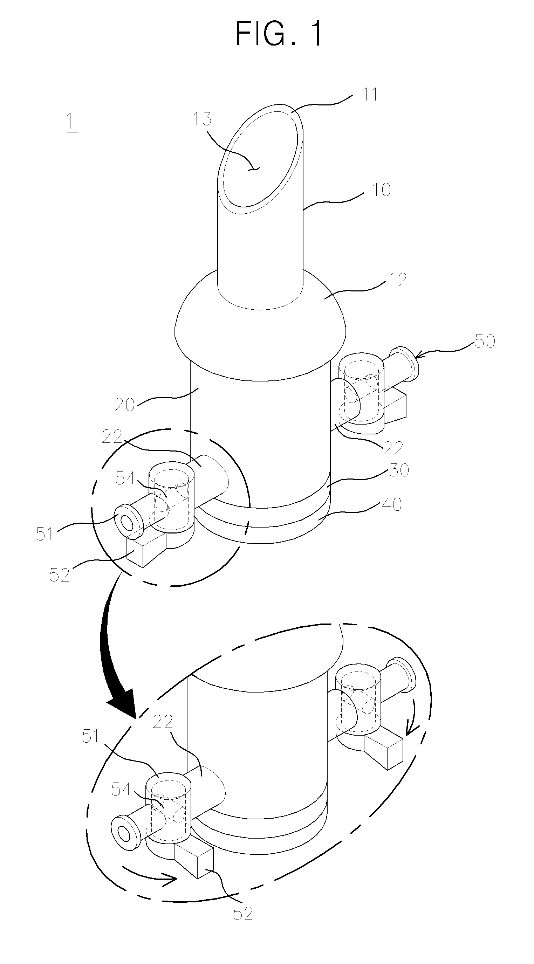

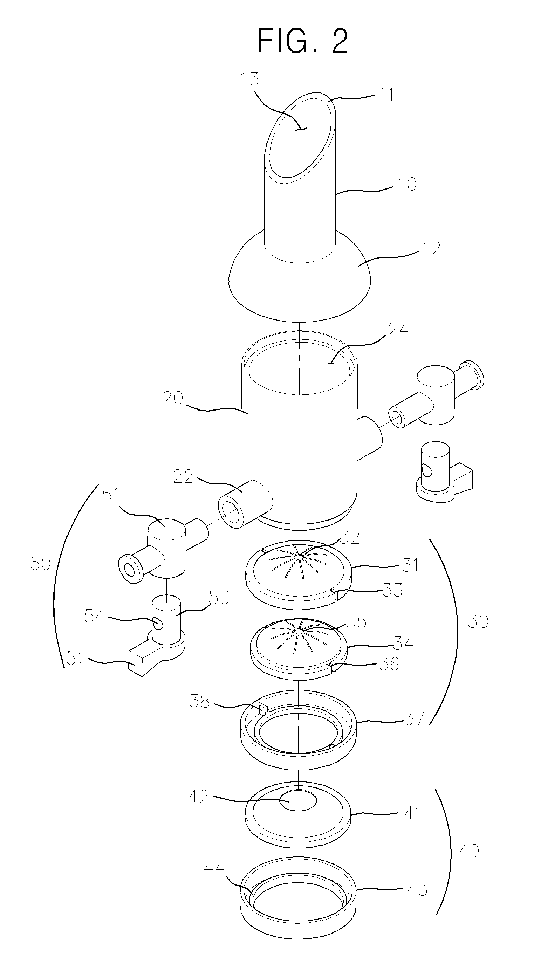

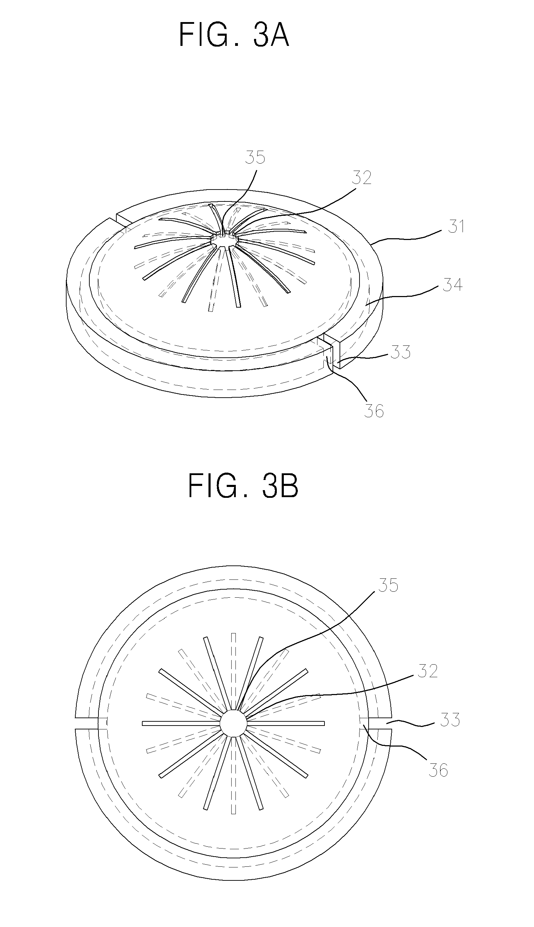

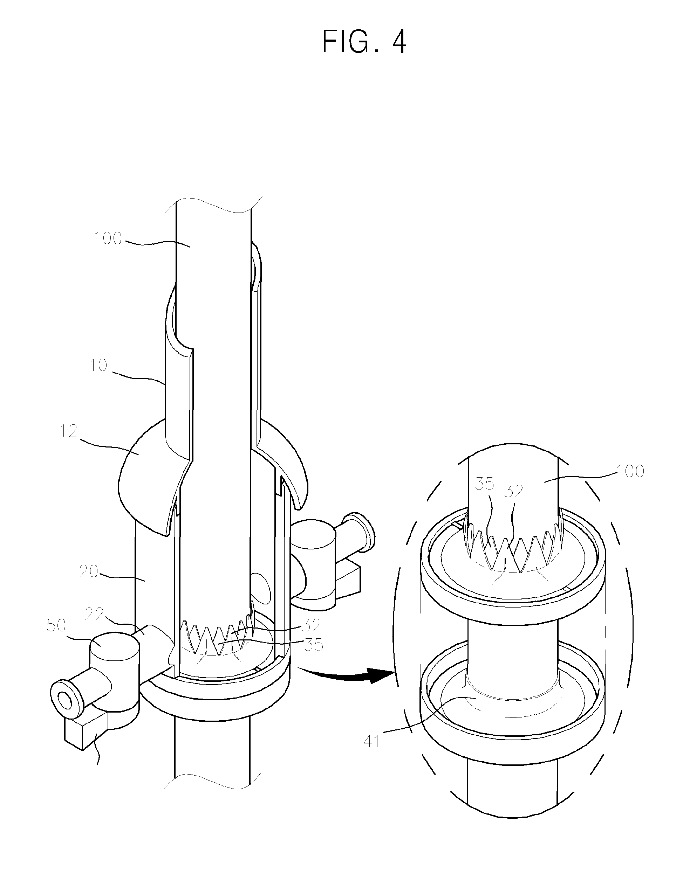

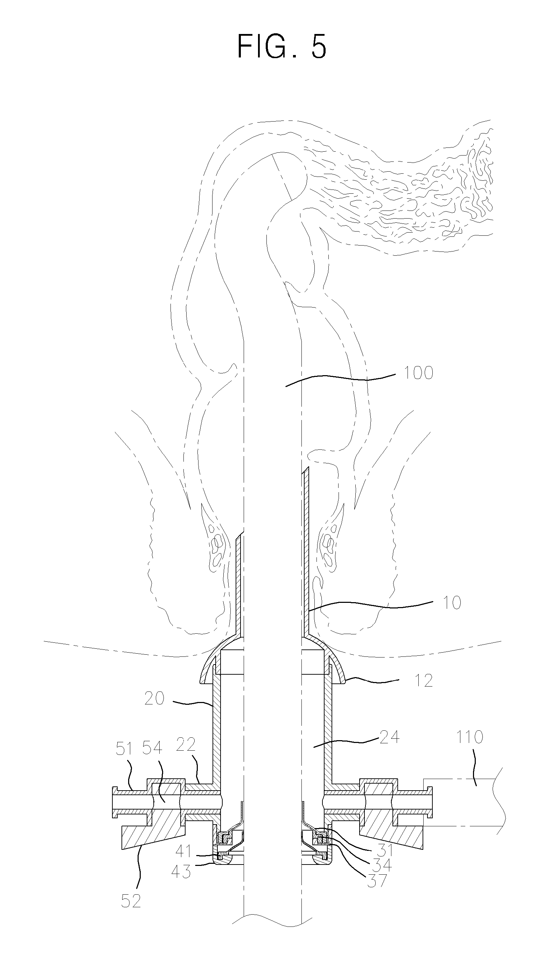

Figure 1 is a perspective view of an auxiliary tool for colonoscopy according to an embodiment of the invention, Figure 2 is an exploded perspective view of an auxiliary tool for colonoscopy according to an embodiment of the invention, Figure 3A and Figure 3B illustrate a first radial leakage prevention membrane and a second radial leakage prevention membrane coupled together, Figure 4 illustrates the leakage prevention structure of a first radial leakage prevention membrane and a second radial leakage prevention membrane, and Figure 5 illustrates an example of utilizing an auxiliary tool for colonoscopy according to an embodiment of the invention.

Illustrated in Figure 1 through Figure 5 are an auxiliary tool 1 for colonoscopy, an anal intubation body 10, an inclined aperture surface 11, an anus cover 12, a hollow part 13, a feces-holding body 20, a holding part 24, a first leakage prevention sub-body 30, a first radial leakage prevention membrane 31, first cut pieces 32, first fitting grooves 33, a second radial leakage prevention membrane 34, second cut pieces 35, second fitting grooves 36, a first ring body 37, fitting protrusions 38, a second leakage prevention sub-body 40, a circular leakage prevention membrane 41, an endoscope through-hole 42, a second ring body 43, a ring-shaped protrusion 44, a valve-connecting part 22, an open/shut valve 50, a socket 51, a valve body 53, an open/shut handle 52, a through-hole 54, an endoscope tube 100, and a suction hose 110.

The auxiliary tool 1 for colonoscopy according to this embodiment can prevent the endoscope tube 100 from directly touching the anus during a colonoscopy examination in spite of its frequent movements, thereby preventing damage to the anal mucosa, etc.

The main components of the auxiliary tool 1 for colonoscopy according to this embodiment includes the anal intubation body 10, which is inserted through the anus, and the feces-holding body 20, which holds fecal matter such as gas, moisture, stool, etc., of the colon inside and prevents it from leaking outside during the colonoscopy examination.

A hollow part 13 may be formed inside the anal intubation body 10, where the inner diameter of the hollow part 13 may be greater than the outer diameter of the endoscope tube 100 generally used.

The outer diameter of the anal intubation body 10 may vary according to the thickness of the main wall, but in one example, the anal intubation body 10 can be formed with a minimal diameter, so as to enable smooth intubation through the anus, within a range that does not hinder the passage of the endoscope tube 100.

At one end of the anal intubation body 10, an inclined aperture surface 11 may be formed, which is an aperture that has its front end inclined at a particular angle with respect to the lengthwise direction of the anal intubation body 10. When the colonoscopy auxiliary tool 1 is inserted through the patient's anus, the inclined aperture surface 11 can be used to achieve smooth intubation with reduced pain incurred to the patient, in spite of the contractions of the anal sphincter muscles.

At the other end of the anal intubation body 10, an anus cover 12 may be formed in an integrated form, where the anus cover 12 may be shaped as a cap having a round curved surface, such as a semispherical shape or the shape of a concave (parabolic) mirror.

When a colonoscopy examination is performed with the anal intubation body 10 inserted into the anus, the anus cover 12 may press against and block the anal opening.

This makes it possible to tightly seal the air inside the colon, so that the colon can be expanded with a minimum amount of air, thereby reducing any pain caused by excessive air injection, and reducing delays in colonoscopy caused by the formation of loops.

Also, when an elderly patient having anal sphincter atony or degraded functioning of the anal sphincter receives a colonoscopy, the air injected inside the colon may leak out through the anus due to the pressure inside the bowels, making it difficult to suitably complete the examination. However, with the present embodiment, the anus cover 12 may block the anus and thus suppress any leakage of air through the anus, regardless of whether or not the sphincter muscles function properly, allowing the surgeon to easily remove polyps in the rectum. Furthermore, any sudden outburst of feces during colonoscopy can be prevented, so that the surgeon may perform the colonoscopy more smoothly.

The anal intubation body 10 and the anus cover 12 may be formed from a transparent material, such as a synthetic resin material, for example. Conventionally, an observation of the lower rectum, such as the anal verge or the anal canal, may require turning the endoscope tube 100 in the reverse direction within the intestine, which creates a risk of puncturing the intestinal wall. Also, friction with the anus caused by frequent movement of the tube may result in severe pain or scars in the anus after the examination. In the present embodiment, however, the area from the anal verge to the lower rectum can be easily observed and diagnosed through the transparent anus cover 12, and since it is possible to adjust the depth by which the endoscope tube 100 is inserted through the anus, the examination can be performed evenly over the area extending from the anal verge to the lower rectum.

The feces-holding body 20 may have one side coupled to the inside of the anus cover 12 and may a holding part 24 provided inside, which connects with the hollow part 13 and which has a greater diameter than the inner diameter of the hollow part 13 of the anal intubation body 10. At the other side of the feces-holding body 20, a leakage prevention body may be installed which suppresses the leakage of the fecal matter held in the holding part 24 outside to the other side.

A curb may be formed at the one side of the inside of the holding part 24 that couples with the anus cover 12, while a ring-shaped protrusion having the same inner diameter as the diameter of the holding part 24 may be formed on an inner side of the anus cover 12 to rest on and couple with the curb of the holding part 24. Of course, this is just one example of a method by which to couple the inner side of the anus cover 12 with one side of the feces-holding body 20, and various other methods of coupling can also be applied.

A leakage prevention body can be formed which allows the endoscope tube 100 to pass through, and since the endoscope tube 100 may pass through the leakage prevention body, the holding part 24 of the feces-holding body 20, and the hollow part 13 of the anal intubation body 10 without directly touching the anus, damage to the anal mucosa, etc., can be avoided in spite of the frequent movement of the endoscope tube 100 during colonoscopy. Also, even after the preparatory procedures for colonoscopy, fecal matter such as stool, etc., may remain within the patient's colon, but the leakage prevention body may prevent such fecal matter from leaking outside in a reverse direction along the path by which the endoscope tube 100 is inserted.

The leakage prevention body may include a first leakage prevention sub-body 30 and a second leakage prevention sub-body 40. For easier explanation and better understanding, the descriptions below will be provided for an example in which a first radial leakage prevention membrane 31, a second radial leakage prevention membrane 34, a circular leakage prevention membrane 41, etc., are all included. However, it is obvious that the leakage prevention body according to this embodiment is not limited to including all of the leakage prevention membranes above and can include only some of the leakage prevention membranes 31, 34, or 41 and their related components.

The first leakage prevention sub-body 30 may include a first radial leakage prevention membrane 31, a second radial leakage prevention membrane 34, and a first ring body 37.

The first radial leakage prevention membrane 31 may include a multiple number of highly flexible first cut pieces 32 that are cut in a radial arrangement, so as to allow flexible opening and shutting. When the endoscope tube 100 is inserted, the first cut pieces 32 may be bent in the movement direction of the endoscope tube 100, as illustrated in Figure 4, due to the insertion pressure, and may thus be opened, all the while keeping close contact with the outer surface of the endoscope tube 100. Thus, the first cut pieces 32 may serve to prevent the fecal matter held in the feces-holding body 20 from leaking out in the reverse direction, while at the same time allowing the endoscope tube 100 to pass through.

The second radial leakage prevention membrane 34 may also include multiple second cut pieces 35 that are cut in a radial arrangement, similarly to the first radial leakage prevention membrane 31, and may thus allow flexible opening and closing, performing the same function as the first radial leakage prevention membrane 31.

Referring to Figure 3, the first radial leakage prevention membrane 31 and the second radial leakage prevention membrane 34 may be coupled such that the first cut pieces 32 and the second cut pieces 35 are staggered, whereby the second cut pieces 35 may be positioned directly underneath the slits between the first cut pieces 32, and the first cut pieces 32 may be positioned directly above the slits between the second cut pieces 35.

The first radial leakage prevention membrane 31 and the second radial leakage prevention membrane 34 may overlap each other and may be coupled fixedly inside the first ring body 37. For coupling the first radial leakage prevention membrane 31 and the second radial leakage prevention membrane 34, there may be fitting protrusions 38 formed on an inner side of the first ring body 37, and there may be first fitting grooves 33 and second fitting grooves 36 formed in the perimeter of the first radial leakage prevention membrane 31 and the second radial leakage prevention membrane 34, respectively. Thus, the first radial leakage prevention membrane 31 and the second radial leakage prevention membrane 34 may be coupled in the proper positions with the first cut pieces 32 and second cut pieces 35 aligned in a staggered configuration, by fitting the fitting protrusion 38 with the first fitting grooves 33 and the second fitting grooves 36.

In this case, when the endoscope tube 100 is inserted and the insertion pressure bends the first cut pieces 32 in the movement direction of the endoscope tube 100, as illustrated in Figure 4, the slits that are widened during this process, as the first cut pieces 32 maintain close contact with the outer surface of the endoscope tube 100, may be blocked by the second cut pieces 35, which are also bent in the movement direction of the endoscope tube 100. Thus, a double barrier may be formed against outside leakage of fecal matter, allowing stronger protection against leakage compared to the case of using only one leakage prevention membrane.

The second leakage prevention sub-body 40 may include a circular leakage prevention membrane 41 and a second ring body 43.

The circular leakage prevention membrane 41 may be configured as a highly flexible membrane in which a through-hole 54 is formed that has a smaller diameter than the outer diameter of the endoscope tube 100. The perimeter of the circular leakage prevention membrane 41 can be fit onto a ring-shaped protrusion 44 formed on a lower surface on the inner side of the second ring body 43, and the second ring body 43 itself can be secured and coupled to a lower portion of the first ring body 37.

When the endoscope tube 100 is inserted, the insertion pressure may bend the membrane 41 around the endoscope through-hole, as illustrated in Figure 4, forming a close contact with the outer surface of the endoscope tube 100. Thus, a triple barrier may be formed that prevents the fecal matter held in the feces-holding body 20 from leaking outside in a reverse direction while allowing the endoscope tube 100 to pass.

In the perimeter of the feces-holding body 20, a valve-connecting part 22 may be formed in an integrated manner to which an open/shut valve 50 may be connected. A suction device can be used to suction any fecal matter that is held in the holding part 24 or remaining in the anal intubation body 10.

If the patient's feces is drawn inside the anal intubation body 10 that guides the endoscope tube 100 during a colonoscopy examination, a suction device can be used to suction and remove the feces. This not only allows the endoscope tube 100 to smoothly pass through inside the anal intubation body 10 but also prevents feces from smearing the inner surface of the anal intubation body 10, thereby providing a clean field of vision for observing the anal verge or the rectum through the transparent anal intubation body 10 and allowing accurate observation and diagnosis.

The open/shut valve 50 that is coupled to the valve-connecting part 22 may be installed between the feces-holding body 20 and a suction hose 110 connected to the suction device, in a manner that allows opening and shutting, so as to control the flow of fluid according to the manipulation of the open/shut handle 52.

The open/shut valve 50 may include a socket 51, in which may be formed an inlet that connects to the valve-connecting part 22 of the feces-holding body 20 and an outlet that connects to the suction hose 110; a cylindrical valve body 53, which has a through-hole 54 formed therein that connects with the inlet and outlet, and which is coupled to the socket 51 in a manner that allows changing directions so as to open and shut the inlet and outlet; and an open/shut handle 52, which connects with the cylindrical valve body 53 and changes the direction of the cylindrical valve body 53 according to a manipulation. The cylindrical valve body 53 and the open/shut handle 52 can be formed as an integrated body, as illustrated in the drawings.

The feces-holding body 20 may be made of a transparent synthetic resin material, so that a surgeon or a nurse may check the amount of fecal matter held in the holding part 24 of the feces-holding body 20 and discharge it at a desired time by turning the open/shut valve 50 on or off. Thus, the fecal matter accumulated during colonoscopy can be easily disposed of without contaminating the examination room.

Also, as the feces-holding body 20 is transparent, the surgeon can see with the unaided eye the depth to which the endoscope tube 100 is inserted, so that the position of the camera on the endoscope tube 100 can be accurately identified, and the examination of the colon can be performed evenly.

While the drawings illustrate an example in which there are two valve-connecting parts 22 formed on the perimeter of the feces-holding body 20, it is also possible to use only one as necessary.

If there are two valve-connecting parts 22 formed, it may be preferable to have them positioned on opposite sides. During a colonoscopy examination, one valve-connecting part 22 can be made to face downward, and the other valve-connecting part 22 can be made to face upward, so as to suction liquid or solid fecal matter through the valve-connecting part 22 facing downward and suction or exhaust gaseous fecal matter (gases) through the valve-connecting part 22 facing upward.

While the invention is described with reference to the embodiments, it will be understood by those skilled in the art that the invention can be modified and changed in various forms without departing from the concept and scope of the invention described in the appended claims. Therefore, it should be understood that the above-mentioned embodiments are exemplary but not definite. The scope of the invention is expressed by the appended claims rather than the above detailed description and it should be understood that the invention includes all the changes and modifications derived from the meanings, scopes, and equivalents of the claims.

## Claims

1. An auxiliary tool for colonoscopy, the auxiliary tool comprising:
an anal intubation body having an aperture surface on one side thereof and having an anus cover on the other side thereof and having a hollow part formed therein, the anus cover shaped as a cap having a round curved surface, the hollow part having a first diameter; and
a feces-holding body having one side thereof coupled to an inner side of the anus cover and having a holding part formed therein and having a leakage prevention body formed on the other side thereof, the holding part having a second diameter and being connected with the hollow part, the leakage prevention body configured to permit passage of an endoscope tube and prevent leakage of fecal matter held in the holding part to the other side.

2. The auxiliary tool of claim 1, wherein the second diameter is greater than the first diameter.

3. The auxiliary tool of claim 1, wherein the leakage prevention body comprises a first leakage prevention sub-body, the first leakage prevention sub-body comprising:
a first radial leakage prevention membrane including a plurality of first cut pieces cut in a radial arrangement, and
a first ring body having the first radial leakage prevention membrane coupled to an inner portion thereof.

4. The auxiliary tool of claim 3, wherein the first leakage prevention sub-body further comprises:
a second radial leakage prevention membrane disposed between the first radial leakage prevention membrane and the first ring body, the second radial leakage prevention membrane including a plurality of second cut pieces cut in a radial arrangement,
and wherein the second radial leakage prevention membrane is coupled in an overlapping manner with the first radial leakage prevention membrane inside the first ring body such that the second cut pieces are positioned directly underneath slits between the first cut pieces.

5. The auxiliary tool of claim 3 or claim 4, wherein the leakage prevention body further comprises a second leakage prevention sub-body, the second leakage prevention sub-body comprising:
a circular leakage prevention membrane having formed therein an endoscope through-hole, the endoscope through-hole having a diameter smaller than an outer diameter of the endoscope tube, and
a second ring body having the circular leakage prevention membrane fitted into an inner portion thereof.

6. The auxiliary tool of claim 1, wherein a valve-connecting part is formed on a perimeter of the feces-holding body,
the auxiliary tool further comprises an open/shut valve installed between a suction device and the valve-connecting part to be capable of opening and shutting, the open/shut valve configured to control a flow of fecal matter according to a manipulation of an open/shut handle, and
the open/shut valve comprises:
a socket having formed therein an inlet connected to the valve-connecting part and an outlet connected to the suction device,
a cylindrical valve body having formed therein a through-hole connecting to the inlet and the outlet, the cylindrical valve body coupled to the socket to be capable of changing direction so as to open and shut the inlet and the outlet, and
an open/shut handle connected to the cylindrical valve body and configured to change a direction of the cylindrical valve body according to a manipulation.

7. The auxiliary tool of claim 1, wherein at least one of the anal intubation body and the feces-holding body is made from a transparent synthetic resin material.
